# EUROPEAN PATENT SPECIFICATION

(11) **EP 1 959 885 B1**
(45) Date of publication and mention of the grant of the patent: **29.07.2015**
(21) Application number: 06824512.5
(22) Date of filing: 06.12.2006
(51) Int. Cl.: A61F 7/10

(54) **DEVICE FOR CONTROLLING THE TEMPERATURE OF LOCAL REGIONS OF A PATIENT'S BODY**
VORRICHTUNG ZUR KONTROLLE DER TEMPERATUR LOKALER REGIONEN DES KÖRPERS EINES PATIENTEN
APPAREIL POUR REGULER LA TEMPÉRATURE DE RÉGIONS LOCALES DU CORPS D'UN PATIENT

(30) Priority: 06.12.2005 SE 0502679
(43) Date of publication of application: 27.08.2008
(73) Proprietor: Dignitana AB, 224 21 Lund (SE)
(72) Inventor: STORMBY, Johan, 217 64 Malmö (SE)
(74) Representative: Andersson, Björn E.
(86) International application number: PCT/SE2006/001383
(87) International publication number: WO 2007/067129

(56) References cited:
- WO-A1-98/16176
- WO-A1-03/022189
- WO-A2-03/072008
- DE-U1- 8 715 804
- NL-C1- 1 025 362
- US-A- 2 502 182
- US-A- 3 000 616
- US-A- 5 879 315
- US-A1- 3 871 381
- US-A1- 2004 127 964
- US-A1- 2005 221 037

## Description

### Background of the Invention

A human being undergoing treatment with cytostatica (chemotherapy) or a similar treatment often suffers of loss of hair. By maintaining during the treatment a low temperature on the hairy region, usually the scalp, such loss can be eliminated or substantially reduced.

US 6 156 059 discloses a head cooler for this purpose comprising a refrigeration unit and a head cover to be placed over a patient's scalp for enclosing the patient's head and neck, flow passages for circulating a fluid coolant supplied by the refrigeration unit, through the device being provided internally of the cover which forms an inside heat exchange surface to be applied against the scalp. A cooling system of this kind for enduring chemotherapy treatment is marketed under the trade mark DigniCap by Dignitana AB, a Swedish company of Malmö, Sweden. The refrigeration unit included in this cooling system has connections for connecting to the unit two separately continuously temperature controlled cooling sections of the cap.

It has been found that also nail changes are a common side effect of systemic chemotherapy, including discoloration, ridging or pitting of the nails and, as the most severe toxic effect in this respect, partial or complete loss of nails or pain in the nail beds of the patient's hands or feet.

US 6 141 801 and WO 99/470 11 describe a glove fitted to a human hand and having a pocket on the palm side or back-hand side of the glove receiving a thermal gelfilled pack adapted for repeated heating and cooling, uncontrolled transmission of heat or cold taking place from the gel-filled pack to other parts of the hand for therapeutic or other purposes. Other hand thermotherapy devices are disclosed in DE 87 15 804 U1.

### Brief Description of the Invention

The present invention relates to device for controlling the temperature of defined local regions of a patient's body.

The object of the invention is to alleviate or eliminate the inconvenience or pain suffered in the nails of hands by a patient undergoing chemotherapy by allocation of the cooling effect to the site or sites from which the pain or other inconvenience emanates i. e. particularly the nail bed on hands, and by accurately controlled local cooling of said site or sites in order to effectively utilize the cooling effect for therapeutic purposes.

This object is achieved by the device defined in claim 1.

### Brief Description of the drawings.

In the accompanying drawings which disclose illustrative embodiments of the method and device according to the invention,
**FIG 1** is a horizontal cross sectional view of a device according to the invention for therapeutic treatment of the hand nails,
**FIG 2** is a vertical cross sectional of the device in FIG 1,
**FIG 3** is vertical cross sectional view of a device for therapeutic treatment of the foot nails, and
**FIG 4** is a horizontal sectional view of the device in FIG 3.

### Detailed Description of the illustrative Embodiments.

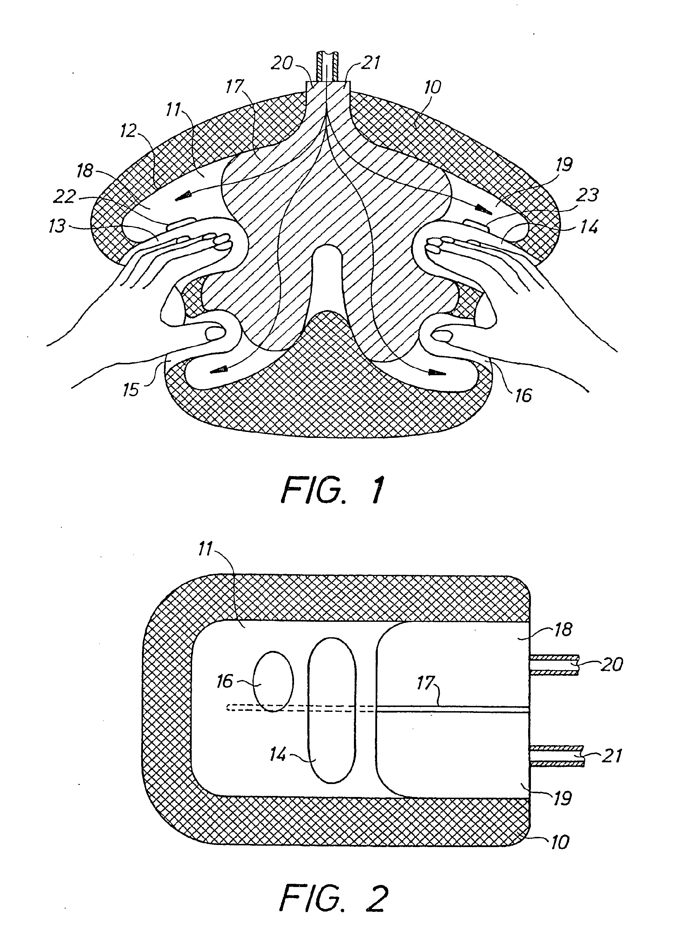

The device shown in FIGS 1 and 2 comprises a body 10 which in its entirety is made of a heat insulating material or includes an inner and an outer shell with a heat insulating material therebetween. An interior space 11 in body 10 is provided with a lining 12 of a fluid impermeable thin and soft material, preferably silicone, the thickness of lining 12 being of the order of 1 or 2 mm. Lining 12 forms two cavities 13 and 14, which open in the outer surface of the body and are adapted to receive the fingers on the left hand and the right hand, respectively, of a patient who shall undergo therapeutic treatment by means of the device, and two cavities 15 and 16 adapted to receive the thumb on the patient's left hand and right hand, respectively. Also these cavities open in the outside surface of the body so that the fingers and the thumbs, respectively, can be inserted into the cavities from the outside of the body. It is the intention that body 10 during treatment of a patient shall be located in the patient's lap with the fingers and thumbs inserted in the associated cavities so that the patient can take a comfortable and relaxed position during the treatment.

The interior space 11 in body 10 is divided by a partition 17 into two compartments 18 and 19 on the upper and lower side of the partition, respectively, compartment 18 being connected to a hose or tube 20 for supply of fluid coolant and compartment 19 being connected to a hose or tube 21 for withdrawal of the coolant from the interior of body 10. Thus, the fluid coolant is forced to circulate through the interior space 11 of body 10 in heat exchanging contact with the heat conducting material 12 cavities 13 to 16 being cooled by the circulating fluid coolant which is delivered from a refrigerating unit and then after passage through the interior of body 10 is returned to the refrigerating unit.

Temperature sensors 22 and 23 are mounted to the outside wall of cavities 13 and 14 and the temperature and flow of the coolant is controlled in dependence of the temperature sensed by these sensors in order to maintain in cavities 13 to 16 the temperature necessary in order to avoid pain and other inconvenience at the nails of the fingers of the patient undergoing therapeutic treatment by means of the device. Further temperature sensors can be provided in the interior space 11 of body 10 at suitable locations therein.

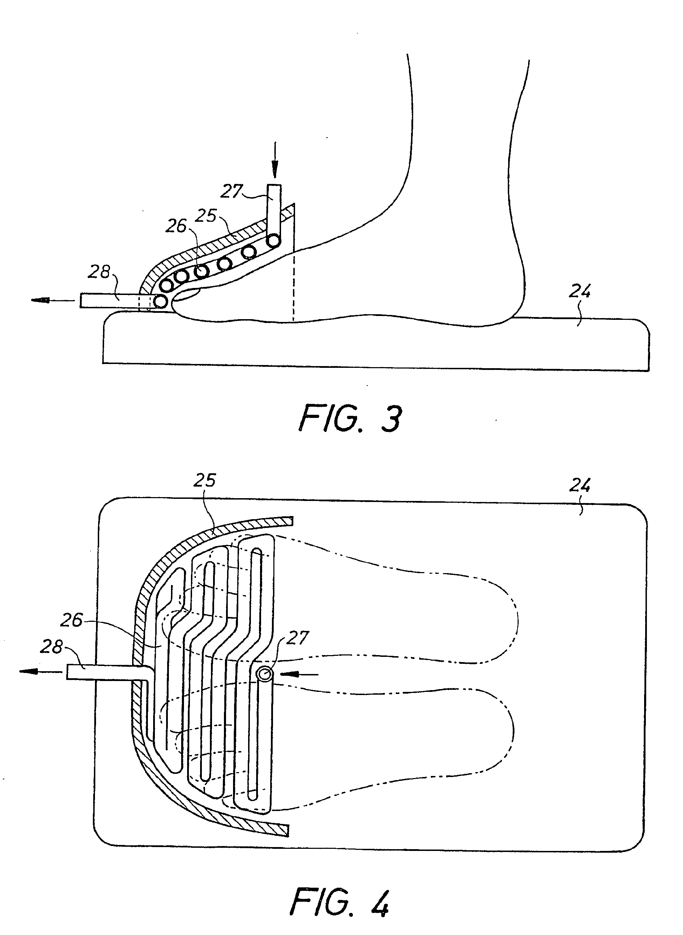

FIGS 3 and 4 disclose a device, not being part of the invention, for treatment of a patient's toes in order to eliminate the severe side effects of chemotherapy. A foot plate 24 of a soft material or alternatively with a soft padding on a rigid substrate is provided with a cover 25 of a heat insulating material. Cover 25 is open at one side thereof so that the toes of a foot can be inserted therein by slipping the foot along the foot plate towards the open side of the cover. Inside the cover there is mounted at the lower side thereof a serpentine shaped tube or hose coil 26 for circulating a fluid coolant, having an inlet 27 and an outlet 28 to be connected to a refrigeration unit in order to maintain inside the cover in the region of the toes on the foot inserted under the cover a predetermined low temperature. One or more temperature sensors can be mounted inside the cover for continuous control of the temperature in said region. If the refrigeration unit is of the kind mentioned above (DigniCap) with connections for two separate cooling sections, two devices with separate temperature control, one for cooling the finger and thumb nails and one for cooling the toe nails, can be connected simultaneously to the refrigeration unit.

## Claims

1. A device for controlling the temperature of local portions of the body of a patient undergoing chemotherapy, comprising a heat insulating body (10), said body (10) being provided with an interior space (11) for flowing fluid coolant through the body, said interior space is lined with a lining (12) of a fluid impermeable material, said lining defining cavities (13-16) opening in the outside surface on the left and right side of the body (10) for receiving therein the fingers on the left hand and the right hand, respectively, of a patient who shall undergo therapeutic treatment by means of the device, said interior space (11) is divided by a partition (17) into two compartments (18, 19) on the upper and lower side of the partition (17), respectively, wherein each of said cavities (13-16) is in contact with the two compartments (18, 19), wherein the two compartments from passages (18,19,26) in said body (10) for flowing a fluid coolant through regions in heat exchanging relation to said cavities, wherein one of the two compartments (18) is connected to a hose or tube (20) for supply of fluid coolant into the interior of the body and the other of the two compartments (19) is connected to a hose or tube (21) for withdrawal of the fluid coolant from the interior of the body, the device further comprising temperature sensors (22,23) mounted to the outside wall of the cavities (13-16).

## Patentansprüche

1. Vorrichtung zur Regelung der Temperatur von lokalen Bereichen eines sich einer Chemotherapie unterziehenden Patienten, umfassend einen wärmeisolierenden Körper (10), welcher Körper (10) mit einem inneren Raum (11) zum Durchfluss eines fluiden Kühlmittels durch den Körper (10) ausgestattet ist, wobei der innere Raum (11) mit einer Auskleidung (12) aus einem fluidundurchlässigen Auskleidungsmaterial ausgekleidet ist, wobei die Auskleidung (12) Vertiefungen (13 - 16) definiert, welche Vertiefungen (13 - 16) sich in einer Außenoberfläche an der linken und rechten Seite des Körpers (10) zur Aufnahme der Finger der linken Hand bzw. rechten Hand eines Patienten, welcher sich einer Chemotherapie mittels der Vorrichtung unterziehen soll, öffnen, wobei der innere Raum (11) über eine Partitionierung (17) in zwei Abteilungen (18, 19) auf der Oberseite bzw. Unterseite der Partitionierung (17) geteilt ist, wobei jede der Vertiefungen (13 - 16) in Kontakt mit den beiden Abteilungen (18, 19) ist, wobei die beiden Abteilungen (18, 19) Durchgänge (18, 19, 26) zum Durchfluss eines fluiden Kühlmittels durch in einer Wärmeaustauschbeziehung mit den Vertiefungen (13 - 16) stehende Bereiche in dem Körper (10) bilden, wobei eine der beiden Abteilungen (18) mit einem Schlauch oder einem Rohr (20) zur Bereitstellung eines fluiden Kühlmittels in das Innere des Körpers (10) verbunden ist und die andere der beiden Abteilungen (19) mit einem Schlauch oder einem Rohr (21) zum Entfernen des fluiden Kühlmittels aus dem Inneren des Körpers (10) verbunden ist, wobei die Vorrichtung ferner auf der Außenwand der Vertiefungen (13 - 16) befestigte Temperatursensoren (22, 23) aufweist.

## Revendications

1. Dispositif de régulation de la température de parties locales du corps d'un patient subissant une chimiothérapie, comprenant un corps thermo-isolant (10), ledit corps (10) étant doté d'un espace intérieur (11) pour l'écoulement de fluide de refroidissement à travers le corps, ledit espace intérieur étant revêtu d'un revêtement (12) d'un matériau imperméable aux fluides, ledit revêtement définissant des cavités (13 à 16) s'ouvrant sur la surface extérieure sur le côté gauche et droit du corps (10) pour y recevoir les doigts de la main gauche et de la main droite, respectivement, d'un patient qui subit un traitement thérapeutique au moyen du dispositif, ledit espace intérieur (11) est séparé par une séparation (17) en deux compartiments (18, 19) sur le côté supérieur et le côté inférieur de la séparation (17), respectivement, dans lequel chacune desdites cavités (13 à 16) est en contact avec les deux compartiments (18, 19), dans lequel les deux compartiments forment des passages (18, 19, 26) dans ledit corps (10) pour l'écoulement d'un fluide de refroidissement à travers les régions en relation d'échange thermique avec lesdites cavités, dans lequel l'un des deux compartiments (18) est raccordé à un tuyau ou un tube (20) pour l'approvisionnement en fluide de refroidissement à l'intérieur du corps et l'autre des deux compartiments (19) est raccordé à un tuyau ou un tube (21) pour l'évacuation du fluide de refroidissement de l'intérieur du corps, le dispositif comprenant en outre des capteurs de température (22, 23) montés sur la paroi extérieure des cavités (13 à 16).
